# EUROPEAN PATENT SPECIFICATION

(11) **EP 4 436 951 B1**
(45) Date of publication and mention of the grant of the patent: **08.04.2026**
(21) Application number: 22899230.1
(22) Date of filing: 16.11.2022
(51) Int. Cl.: A61K 31/223, A61P 9/12, A61P 9/00, A61K 38/05, A61K 9/00, A61K 47/20

(54) **USE OF AURANTIAMIDE ACETATE IN THE TREATMENT OF HYPERTENSION**
VERWENDUNG VON AURANTIAMIDACETAT BEI DER BEHANDLUNG VON HYPERTONIE
UTILISATION D'ACÉTATE D'AURANTIAMIDE DANS LE TRAITEMENT DE L'HYPERTENSION

(30) Priority: 23.11.2021 TR 202118240
(43) Date of publication of application: 02.10.2024
(73) Proprietor: Akdeniz Universitesidoner Sermaye Isletme Mudurlugu, Antalya (TR)
(72) Inventor: ASLAN, Mutay Aydin, 07070 Kampus Antalya (TR); BASRALI, Filiz, 07070 Kampus Antalya (TR); ULKER, Pinar, 07070 Kampus Antalya (TR)
(74) Representative: Sevinç, Cenk
(86) International application number: PCT/TR2022/051305
(87) International publication number: WO 2023/096617

(56) References cited:
- CN-A- 1 363 273
- CN-A- 103 508 922
- JP-A- H0 873 352
- US-A1- 2010 272 709
- ISSHIKI KENGO ET AL: "Aurantiamide Acetate, a Selective Cathepsin Inhibitor, Produced by Aspergillus penicilloides", BIOSCIENCE, BIOTECHNOLOGY, AND BIOCHEMISTRY, vol. 65, no. 5, 1 January 2001 (2001-01-01), pages 1195 - 1197, XP093071171, DOI: 10.1271/bbb.65.1195
- IRANSHAHY MILAD ET AL: "A review of traditional uses, phytochemistry and pharmacology ofPortulaca oleraceaL", JOURNAL OF ETHNOPHARMACOLOGY, ELSEVIER IRELAND LTD, IE, vol. 205, 8 May 2017 (2017-05-08), pages 158 - 172, XP085063520, ISSN: 0378-8741, DOI: 10.1016/J.JEP.2017.05.004
- ASLAN M. ET AL: "P-01.4-006 - Effects of asperglaucide on endothelium function in 2K1C hypertensive rats", vol. 12, no. S1, 1 July 2022 (2022-07-01), US, pages 67 - 336, XP093241690, ISSN: 2211-5463, Retrieved from the Internet <URL:https://onlinelibrary.wiley.com/doi/pdf/10.1002/2211-5463.13440> DOI: 10.1002/2211-5463.13440
- ASLAN MUTAY ET AL: "Effects of aurantiamide on a rat model of renovascular arterial hypertension", vol. 475, no. 10, 15 August 2023 (2023-08-15), Berlin/Heidelberg, pages 1177 - 1192, XP093241609, ISSN: 0031-6768, Retrieved from the Internet <URL:https://link.springer.com/article/10.1007/s00424-023-02850-8/fulltext.html> DOI: 10.1007/s00424-023-02850-8
- YOON CHI-SU; KIM DONG-CHEOL; LEE DONG-SUNG; KIM KYOUNG-SU; KO WONMIN; SOHN JAE HAK; YIM JOUNG HAN; KIM YOUN-CHUL; OH HYUNCHEOL: "Anti-neuroinflammatory effect of aurantiamide acetate from the marine fungusAspergillussp. SF-5921: Inhibition of NF-κB and MAPK pathways in lipopolysaccharide-induced mouse BV2 microglial cells", INTERNATIONAL IMMUNOPHARMACOLOGY, vol. 23, no. 2, 16 October 2014 (2014-10-16), NL , pages 568 - 574, XP029096836, ISSN: 1567-5769, DOI: 10.1016/j.intimp.2014.10.006
- RAMESH SUHAS; DASE CHANNE GOWDA: "Structure‐Based Rationale Design and Synthesis of Aurantiamide Acetate Analogues – Towards a New Class of Potent Analgesic and Anti‐inflammatory Agents", CHEMICAL BIOLOGY & DRUG DESIGN, vol. 79, no. 5, 13 February 2012 (2012-02-13), Hoboken, USA, pages 850 - 862, XP072378356, ISSN: 1747-0277, DOI: 10.1111/j.1747-0285.2012.01331.x
- ISSHIKI KENGO, YASUYUKI ASAI,SUMIKO TANAKA,MAKI NISHIO,TOMOFUMI UCHIDA,TORU OKUDA,SABURO KOMATSUBARA , NAOKI SAKURAI: "Aurantiamide Acetate, a Selective Cathepsin Inhibitor, Produced by Aspergillus penicilloides", BIOSCIENCE, BIOTECHNOLOGY, AND BIOCHEMISTRY, vol. 65, no. 5, 1 January 2001 (2001-01-01), pages 1195 - 1197, XP093071171, DOI: 10.1271/bbb.65.1195
- YANG YANG, DAI JIA-HE, SHI CHONG-YIN, TAO LIANG, HE SHUI-LIAN, TIAN YANG, SHENG JUN: "Identification of novel targets of aurantiamide acetate against osteoporosis through in silico and in vitro approaches", MATERIALS EXPRESS, vol. 10, no. 12, 1 December 2020 (2020-12-01), pages 2056 - 2062, XP093071174, ISSN: 2158-5849, DOI: 10.1166/mex.2020.1851

## Description

### Technical Field of the Invention

The present invention relates to the use of aurantiamide acetate in the treatment of hypertension. Aurantiamide acetate is used in the preparation of blood pressure lowering drugs or health care products.

### State of the Art

The highest pressure on the artery wall when blood is pumped forms systolic blood pressure (systolic blood pressure), and the lowest pressure during relaxation forms the diastolic blood pressure (diastolic blood pressure). Although the optimum blood pressure rate varies from person to person, the ideal is between 90/60 mmHg and 120/80 mmHg (systolic blood pressure between 90-120, diastolic blood pressure between 60-80). The blood pressure value measured above the optimum value is defined as high blood pressure. Hypertension, which means high blood pressure, is one of the leading preventable risk factors for various disorders such as heart failure, heart attack, some rhythm disorders such as atrial fibrillation, and chronic kidney diseases. Therefore, it is an important topic that blood pressure (blood pressure) is within the normal range for maintaining healthy body functions. Hypertension is classified into various stages according to the measured blood pressure values. Blood pressure values between 140/90 mmHg and 159/99 mmHg are defined as stage 1 hypertension, blood pressure values between 160/100 mmHg and 179/109mmHg are defined as stage 2 hypertension, and a systolic blood pressure of 180 mmHg and a diastolic blood pressure of 110 mmHg or more are defined as stage 3 hypertension. Acute Severe hypertension is used for conditions that have exceeded 180/110 mmHg and are now life-threatening where high blood pressure results in clinical events that damage organs. High blood pressure is a common and important health problem seen in one out of every three people in our country. In developed countries, the lifetime risk of developing hypertension is approximately 90%. The most common symptoms are dizziness, headache, heart pain, tinnitus, shortness of breath, double or blurred vision, nosebleeds, and irregular heartbeats.

Hypertension can be easily controlled with medication, exercise, and proper nutrition. However, if left untreated, it causes serious problems such as heart failure, heart enlargement, narrowing of the arteries, stroke, kidney failure, and blindness.

After the diagnosis of hypertension is made, different treatment programs are applied according to the hypertension level of the person. First of all, there are some factors that people need to change in their daily lifestyle. For example; since salt use is one of the factors that affect blood pressure the most, salt consumption should be definitely reduced and regular exercise is recommended. In addition thereto ,unhealthy foods are listed and determined as prohibited foods. It is recommended to quit habits such as smoking and alcohol. If deemed necessary, psychological support is provided for individuals living under intense stress. People with advanced disease are treated with antihypertensive drugs. These people enter a long-term treatment period. For some patients, treatment may even last a lifetime. Antihypertensive drugs lower blood pressure by dilating blood vessels, that is, by preventing them from narrowing or contracting, or by reducing the workload of the heart. It is known in the state of the art that drugs such as diuretics, alpha blockers, beta blockers, vasodilators, ACE inhibitors (angiotensin converting enzyme inhibitors), angiotensin 2 receptor blockers (ARBs), calcium channel blocker, or central adrenergic inhibitor are used in the treatment of hypertension. The most common combinations of these drugs are ACE inhibitors with thiazide diuretics (monopril plus, coversyl plus, etc.), thiazide diuretics with angiotensin 2 receptor blockers, or potassium-sparing diuretics (karvezide, co-diovan, etc.), beta-blockers with thiazide diuretics (tenoretic etc.), thiazide diuretics with potassium-sparing diuretics, and ACE inhibitors with calcium channel blockers. Said hypertensive drugs have side effects such as allergic reaction, low blood pressure (hypotension), arrhythmia (abnormal heart rhythm), palpitations (irregular and felt heartbeats), dizziness, blackouts, syncope, headache, severe sunburn, increased sensitivity to sunlight leading to rash (photosensitivity), weakness, fatigue, abnormal bleeding, abdominal discomfort, nausea, diarrhea, abnormal color change, depression, constipation, and joint or lower back pain. Approximately 35% of patients who are started on antihypertensive medication with the diagnosis of hypertension quit taking their drugs within the first 6 months, and the most important reason thereof is drug side effects and patient dissatisfaction [1].

The aurantiamide acetate compound with the molecular formula of C₂₇H₂₈N₂O₄ and a molecular weight of 444.531 g/mol has the molecular structure indicated by Formula 1. This compound belongs to the class of organic compounds known as phenylalanine and its derivatives. These are compounds containing phenylalanine or a derivative thereof, resulting from the reaction of phenylalanine at the amino group or carboxy group, or the replacement of any hydrogen of the glycine with a heteroatom [2]. Aurantiamide acetate has anti-cancer, anti-inflammatory, and antinociceptive activities and suppresses the growth of malignant gliomas by blocking autophagic efflux. In addition, inhibits cysteine proteinases, particularly cathepsin L (3.4.22.15) and B (3.4.22.1), and it has an anti-neuroinflammatory effect on LPS stimulation through inhibition of NF-κB, JNK, and p38 pathways [3].

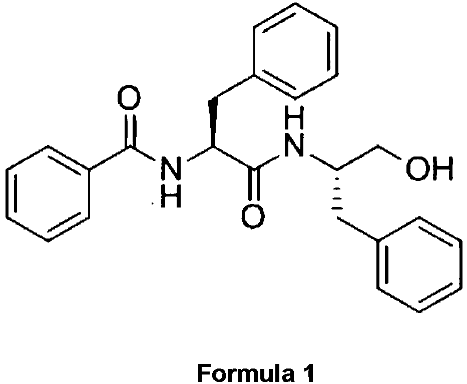

In a prior art study, the pharmacokinetics and biodistribution of aurantiamide and aurantiamide acetate were investigated by oral administration of Portulaca oleracea L extracts in rats. Mass spectrometric measurements were performed to examine the concentrations of aurantiamide and aurantiamide acetate in plasma and their distribution in various organs in rats. As a result of this study, it was observed that aurantiamide and aurantiamide acetate were rapidly absorbed after administration. Both compounds reached their maximum plasma concentration in approximately 12 minutes. A decrease of approximately 90% in concentrations occurred 4 hours after administration, and there was no long-term accumulation in tissues. No toxic effects were observed in administrations up to 100mg/kg.

The patent application numbered CN106431960A discloses the application of aurantiamide acetate in the preparation of an anti-influenza virus medicine, and the clinical prevention and amelioration of influenza by this drug. Also, the above-mentioned application discloses a method for separating aurantiamide acetate from the Radix Isatidis plant. Aurantiamide acetate has been shown to have an effect of resisting influenza virus and an inhibiting effect on the cytopathic effect mediated by influenza virus.

The patent application numbered KR20160045980A discloses a composition for the preventing and treating neurodegenerative diseases comprising aurantiamide acetate as an active ingredient. Particularly, it is disclosed a composition for preventing and treating neurodegenerative diseases comprising aurantiamide acetate separated from Aspergillus SF-5921 and having an anti-neuritic effect as an active ingredient. The composition for preventing and treating neurodegenerative diseases comprising aurantiamide acetate as active ingredient is effective to inhibit the production of nitrogen oxides, the expression of PGE-2 and IL-1b, INOS, and COX-2. In addition, it is effective in suppressing the activation of NF-KB, lowering the level of IKK α/β/γ phosphorylation and lowering the level of JNK and p38 MAPK phosphorylation, and it has no cytotoxicity.

The patent application numbered JPH0873352A discloses a cathepsin B inhibitor for use in the treatment of hypertension. The document describes an epoxy succinamic acid derivative or a pharmaceutically acceptable salt thereof as a therapeutic agent, which may be administered orally. JPH0873352A addresses the treatment of hypertension by providing a pharmacological agent that may act on the renin-angiotensin system through inhibition of angiotensin-converting enzyme (ACE).

The patent application numbered US2010/272709A1 relates to the treatment of diseases associated with cathepsin- or dynamin-related mechanisms. The document particularly addresses conditions characterized by proteinuria, kidney disease, complications related to hypertension and cognitive disorders, and discloses the use of cathepsin L inhibitors as therapeutic agents. US2010/272709A1 describes the prevention or reduction of podocyte damage as a means to treat diseases such as focal segmental glomerulosclerosis, diabetic nephropathy and IgA nephropathy, as well as other conditions in which cathepsin activity plays a role.

The patent application numbered CN1363273A describes a pharmaceutical invention published in China on 14 August 2002 by Yan Qinghui. The document relates to therapeutic compounds or pharmaceutical compositions for use in a medical context. CN 1 363 273 A is cited to represent the general state of the art in the technical field of pharmaceutical treatments.

Said hypertensive drugs have side effects such as allergic reaction, low blood pressure (hypotension), arrhythmia (abnormal heart rhythm), palpitations (irregular and felt heartbeats), palpitations (irregular and felt heartbeats), dizziness, blackouts, syncope, headache, severe sunburn, increased sensitivity to sunlight leading to rash (photosensitivity), weakness, fatigue, abnormal bleeding, abdominal discomfort, nausea, diarrhea, abnormal color change, depression, constipation, and joint or lower back pain.

### Brief Description and Objects of the Invention

The present invention relates to the use of aurantiamide acetate in the treatment of hypertension. The present invention relates to the use of aurantiamide acetate in the preparation of blood pressure lowering drugs or health care products. The present invention discloses the use of aurantiamide acetate in the treatment of hypertension, particularly its restoration of endothelium-mediated vascular function in hypertension. The references to methods of treatment in this description are to be interpreted as references to the compounds, pharmaceutical compositions and medicaments of the present invention for use in a method for treatment of the human (or animal) body by therapy.

The main object of the present invention is to provide an antihypertensive drug with low side effects for the effective treatment of hypertension patients. An effective treatment is provided in hypertension patients with the endothelium-mediated vascular function-regulating effect of aurantiamide acetate and reducing the pressure in the vessels by increasing blood flow. In addition, it is ensured that patients do not stop using their drugs and that the treatment is not interrupted by means of the absence of side effects such as allergic reaction, low blood pressure (hypotension), arrhythmia (abnormal heart rhythm), palpitations (irregular and felt heartbeats), palpitations (irregular and felt heartbeats), dizziness, blackouts, syncope, headache, severe sunburn, increased sensitivity to sunlight leading to rash (photosensitivity), weakness, fatigue, abnormal bleeding, abdominal discomfort, nausea, diarrhea, abnormal color change, depression, constipation, and joint or lower back pain.

Another object of the present invention is to reduce the complications of hypertension and cardiovascular morbidity in hypertension patients. By means of the present invention, said complications and thus morbidity are reduced by using aurantiamide acetate in the treatment of hypertension, which has an endothelium-mediated vascular function-regulating effect in hypertension.

Yet another object of the present invention is to increase the blood flow and reduce the pressure in the vessels in patients with hypertension. In the present invention, erythrocyte deformability is increased by the administration of aurantiamide acetate. Thus, erythrocytes can change their shape as they pass through small vessels and increase fluidity of blood by lowering blood viscosity.

The present invention provides an antihypertensive drug that improves endothelium-mediated vascular function for use in the treatment of hypertension.

### Description of the Figures

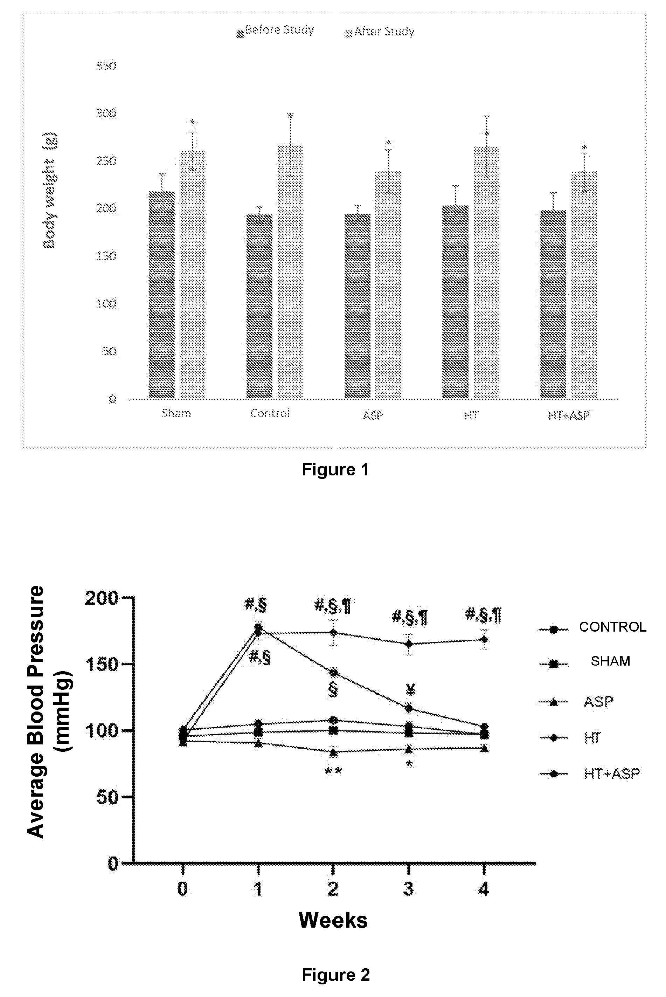

**Figure** 1 Bar graph showing changes in body weight in experimental groups during the administration period to observe the effect of the aurantiamide acetate of the present invention on the change in body weight. (ASP aurantiamide acetate-treated group; HT, hypertensive group; *, p<0.001, compared with pretreatment).
**Figure** 2 Graph showing mean arterial blood pressures in experimental groups during the administration period in order to observe the effect of the aurantiamide acetate of the present invention on the change in mean arterial blood pressures. (ASP, aurantiamide acetate-treated group; HT, formed hypertension group with 2K-1C model; HT+ASP, the group in which aurantiamide acetate treatment was applied after hypertension was established with the 2K-1C model; **, p<0.05, comparison with SHAM and control groups; *, p<0,05, comparison with the control group; #, p<0,001, comparison with week 0 values; §, p<0,001, comparison with control, SHAM and ASP groups; ¶, p<0,01, comparison with HT+ASP group)

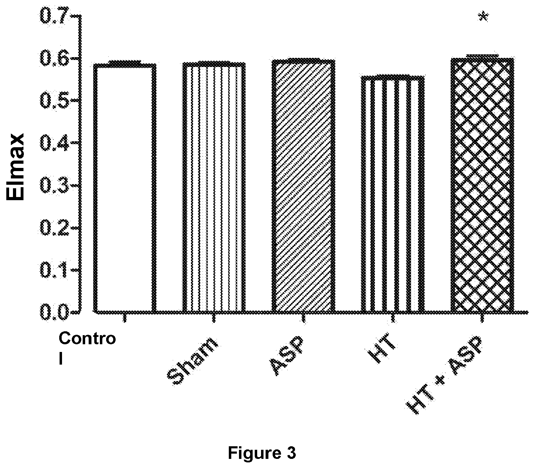

**Figure** 3 Graph showing the erythrocyte deformability measurement results during the application period in the test groups, Elmax values to observe the effect of aurantiamide acetate, which is the subject of the invention, on the change in erythrocyte deformability (ASP, aurantiamide acetate-treated group; HT, formed hypertension group with 2K-1C model; HT+ASP, the group in which aurantiamide acetate treatment was applied after hypertension was established with the 2K-1C model; *, p<0.05, comparison with HT group)

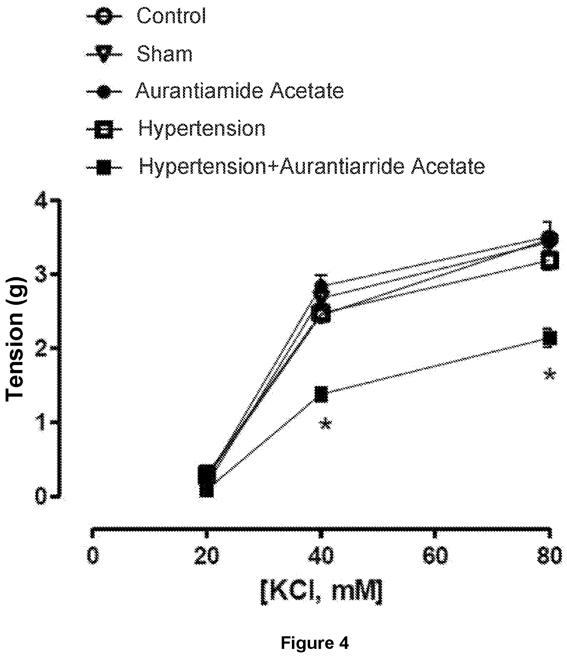

**Figure 4** Dose-response curve of potassium chloride (KCl)-mediated contraction of the Thoracic Aorta (*, p<0.001, difference from hypertension)

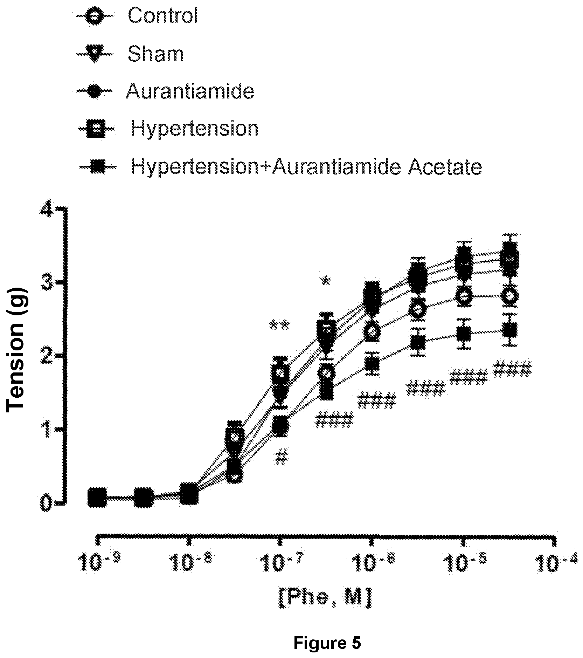

**Figure 5** Dose-response curve of phenylephrine (Phe)-mediated contraction of the Thoracic Aorta (*, p<0.05 difference from control group; **, p<0.01 difference from control group; p<0.05 difference from the hypertension group; ###, p<0.001 difference from hypertension group)

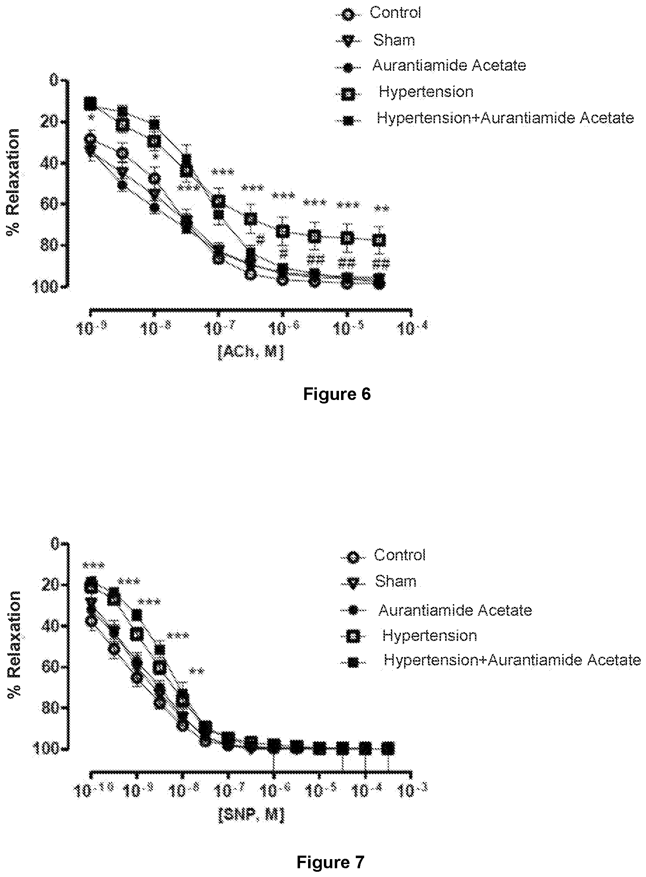

**Figure 6** Dose-response curve of acetylcholine (ACh)-mediated relaxation of the Thoracic Aorta (*, p<0.05 difference from control group; **, p<0.01 difference from the control group; ***, p<0.001 difference from control group; #, p<0.05 difference from hypertension group; ##, p<0.01 difference from hypertension group)
**Figure 7** Dose-response curve of sodium nitroprusside (SNP)-mediated relaxation of the Thoracic Aorta (**, p<0.01 difference from control group; ***, p<0.001 difference from control group)

### Detailed Description of the Invention

The present invention relates to the use of aurantiamide acetate in the treatment of hypertension. Aurantiamide acetate, which is used in the preparation of blood pressure-lowering drugs or health care products, improves endothelium-mediated vascular function in hypertension, thereby being particularly effective in the treatment of hypertension.

For the effective treatment of hypertension patients with aurantiamide acetate, an effective treatment is provided in patients with hypertension by means of an antihypertensive drug with low side effects and by means of the aurantiamide acetate's regulating effect on endothelium-mediated vascular function and reducing the pressure in the vessels by increasing blood fluidity. erythrocyte deformability is increased by the application of aurantiamide acetate. Thus, erythrocytes can change their shape as they pass through small vessels, reducing blood viscosity, and increasing blood fluidity. In addition, it does not have side effects including allergic reaction, low blood pressure (hypotension), arrhythmia (abnormal heart rhythm), palpitations (irregular and felt heartbeats), dizziness, blackouts, syncope, headache, severe sunburn, increased sensitivity to sunlight leading to rash (photosensitivity), weakness, fatigue, abnormal bleeding, abdominal discomfort, nausea, diarrhea, abnormal color change, depression, constipation, and joint or lower back pain.

5 experimental groups were formed to observe the effects of aurantiamide acetate. Eight four-week-old male Wistar rats were used in each group. The experimental groups created are as follows:
1. Control group,
2. SHAM operated (the abdominal cavity is opened and closed by suturing again without any procedure) group,
3. Aurantiamide acetate (ASP) administered group,
4. Hypertension (HT) group,
5. Hypertension+aurantiamide acetate (HT+ASP) administered group.

Hypertension was induced in rats with the Goldblatt model with two kidneys and a single clip (2K-1C). In this model, no operation was performed on the right kidney. The left renal artery was narrowed to 0.22 mm in diameter. In the surgical hypertension model created, renin secretion by the kidney with decreased blood flow led to increased angiotensin II (ANG II) formation and therefore an increase in blood pressure. Sodium excretion by the intact contralateral kidney increases as blood pressure increases (pressure natriuresis), therefore, there was no sodium retention. The rats were anesthetized intraperitoneally before the operation. In the creation of the hypertension model, 90 mg/kg ketamine and 10 mg/kg xylazine were administered intraperitoneally for anesthesia. After anesthesia, the abdomens of the rats were shaved and disinfected with betadine. After disinfection, a midline incision was made and the left kidney was exposed. The left renal artery was isolated from the renal vein, and to induce 2K-1C renovascular hypertension, the circumference of the left renal artery was narrowed gently to 0.22 mm in diameter by 5/0 non-absorbable silicone-coated silk sutures The same procedure was applied to SHAM operated group rats, but the left renal artery was not narrowed. Postoperatively, the peritoneum was sutured with 3/0 absorbable polyglycan suture and the skin layer was sutured with 3/0 non-absorbable silk suture. In the hypertension model, it was waited for 1 week to observe the increase in blood pressure in the hypertension and hypertension+aurantiamide acetate groups, and then blood pressure was measured.

The blood pressures of the animals were measured by the non-invasive tail-cuff method. An occlusive cuff and a piezoelectric impact sensor are placed around the tail. Arterial blood pressure measurements were taken weekly at the same time of day (9:00 am to 12:00 am) to avoid influencing the circadian cycle. Basal blood pressure was measured after delivery of each rat, and blood pressures were recorded regularly every week for 4 weeks until sacrifice. The data acquisition system reported blood pressure (mmHg) as systolic, diastolic, and mean. Each measurement was repeated three times and the average of these three data was taken.

Aurantiamide acetate was applied to the ASP and **HT+ASP** groups among the experimental groups. A 100 mM stock was prepared by dissolving 10 mg of aurantiamide acetate in 225 µl of dimethyl sulfoxide (DMSO). For 200 g rat, µl of aurantiamide acetate was administered in 100 µl. Weekly 2.5 mg/kg of aurantiamide acetate was administered intraperitoneally for 4 weeks, 0.5 mg/kg daily, 5 days a week. In total, each rat was given 10 mg/kg of aurantiamide acetate. To eliminate the effects of daily intraperitoneal (i.p.) injection; equal volumes (100 µl) of i.p.DMSO were injected into the rats of control group, SHAM-operated group, and hypertension group. Changes in body weight, blood pressure values, erythrocyte deformability, vascular isolation, and vascular tone were investigated in the experimental groups during the experiment.

Changes in body weight were observed in the experimental groups during the experimental period. The initial weights of the rats were measured in the range of 180-200 g. The weights of each rat before the experiment (Day 0) and after the experiment (at the end of the 4th week) were recorded. Results are shown in Figure 1 as mean ± standard deviation (SD). In all experimental groups, the body weight measured after the 4th week showed a significant increase compared to the pre-experiment. There was no significant difference in body weights before and after between the experimental groups.

Blood pressure values were examined in the experimental groups during the experimental period. The mean arterial blood pressure values of the groups are shown in Figure 2. The basal (Week 0) blood pressure did not differ between the groups (p>0.05). Blood pressure values did not show a significant difference within the control, SHAM, and ASP groups according to the weeks (p>0.05). Similarly, when the control and SHAM groups were compared, no significant difference was found between blood pressures throughout the study (p>0.05). The blood pressure values measured in the second week in the ASP group were significantly lower than the control and SHAM operated groups (**, p<0.05). The blood pressure values measured in the third week in the ASP group were significantly lower than the control group individually (*, p<0.05). When the **HT** group is evaluated within itself, after applying the 2K-1C model, blood pressure values measured at weeks 1,2, 3, and 4 are significantly higher than the value at week 0 (#, p<0.001). When the HT group was compared with the other groups, it has been shown that blood pressure values measured at weeks 1, 2, 3, and 4 are significantly different compared to the control, SHAM, and ASP groups (§, p<0.001). Compared to the hypertension group, at the first measurement (1st week) following the establishment of renal hypertension in the HT+ASP group, no difference was observed in blood pressure (p<0.05), however, there was a significant difference between control, SHAM, and ASP groups (§, p<0.001). After initiation of ASP treatment in hypertensive rats, the blood pressures measured in the second week in the HT+ASP group showed a significant decrease compared to the HT group (¶ p<0.001). The blood pressures measured in the second week in the HT+ASP group were found to be significantly higher than the control, SHAM, and ASP groups (§, p<0.001). When the blood pressures measured at the third week in the HT+ASP group were evaluated, again, a significant decrease was observed compared to the HT group (¶ p<0.001), blood pressure values were found to be significantly higher than only the ASP group (¥, p<0.001). When the blood pressures measured in the last week (week 4) in the HT+ASP group are evaluated, again, a significant decrease was observed compared to the HT group (¶ p<0.001). In addition, blood pressure values were reduced to basal levels and no difference was observed between the control, SHAM, and ASP groups (p>0.05).

Erythrocyte deformability was measured in the experimental groups. An ektacytometer was used for erythrocyte deformability. Erythrocyte deformability was evaluated by laser diffraction analysis at various fluid shear forces. The erythrocytes were suspended at a dilution of about 1/200 in a solution of polyvinylpyrrolidone (PVP-360, MW 360 kD) prepared at a density of 5% and a viscosity of 23 in isotonic phosphate buffer. After the suspension was placed in the system, it was rotated at a speed to create the appropriate shear forces and was left under the influence of these forces. The beam emanating from a laser source in the system reached the erythrocyte suspension and formed a diffraction pattern reflected on the screen. This pattern reflected the shape of the erythrocytes in suspension and their orientation to the flow created by the rotational motion. Thus, the deformability of erythrocytes in response to increased shear forces was determined. Measurements were carried out at 37°C. The erythrocyte deformability was calculated by the system as an elongation index. Elongation index (EI) values were measured between nine shear forces (0.30 - 75.02 Pa). EI=(L-W)/(L+W), wherein L and W are the length and width of the diffraction pattern. An increased El at a given shear stress indicates greater cell deformation and thus greater erythrocyte (RBC) deformability. The erythrocyte deformability measurement results are given in Figure 3. When the erythrocyte deformability data of the groups were compared; no difference was observed between the Elmax values of the control, SHAM, and aurantiamide acetate groups (p>0.05). In the hypertension group, the Elmax value decreases compared to the control group, even if it is not significant (p>0.05). Considering the hypertension + aurantiamide acetate group, a significant increase in erythrocyte deformability was also observed compared to the hypertension group (*, p<0.01, compared with the hypertension group). The increased deformability in the treatment group compared to the hypertension group enabled erythrocytes to change shape as they pass through small vessels and an increase in blood fluidity by lowering the blood viscosity.

Vascular isolation was investigated in the experimental groups. The rats in all experimental groups were anesthetized with intraperitoneal ketamine/xylazine (45 mg/kg ketamine and 10 mg/kg xylazine) anesthesia at the end of the 4-week experimental period. After their abdomens were opened, blood was drawn from their abdominal aorta to obtain plasma as well as for use in measuring deformability, and rats were sacrificed by anemia. The thoracic aorta was quickly opened and the thoracic aorta was separated from the other tissues, and removed. As soon as the thoracic aorta tissue is removed, it was taken into a beaker containing Krebs solution (110 mM sodium chloride (NaCl), 5 mM potassium chloride (KCI), 24 mM sodium bicarbonate (NaHCO₃), 1 mM mono potassium phosphate (KH₂PO₄), 1 mM magnesium sulfate heptahydrate (MgSO₄.7H₂O), 2.5 mM calcium chloride (CaCl₂), 10 mM glucose, 0.02 mM ethylenediamine tetraacetic acid (EDTA); pH=7.4). After the tissue was washed with cold Krebs, the dissection stage was started. For the microdissection stage, the thoracic aorta, taken in a petri dish containing cold Krebs, is fixed to the medium, and fat and connective tissue were removed from the artery at the appropriate magnification by using a dissecting microscope. The isolated thoracic aorta is divided into rings of appropriate length. In addition, the rest of the aorta tissue was removed and stored under appropriate conditions for histological and biochemical parameters. Vascular tone was evaluated in the experimental groups. Vascular tone in the experimental groups was analyzed as in the previous work of the project team. After the isolated thoracic aorta is divided into 2 - 2.5 mm long rings; it was gasified with a gas mixture containing 95% O₂ - 5% CO₂, and it was placed in an organ bath with 10 ml of Krebs solution at 37°C and pH 7.4. After the vessel rings are attached to the isometric transducer by means of a tight rope by hanging them on steel wires, they were washed every 15 minutes under 1 gram relaxation tension and rested for a total of 60 minutes. If the tension changed after each wash, attention was paid to bring it back to 1 gram of tension. After a one-hour relaxation tension (rest period), the vitalization stage, which is the prepulse stage, was applied to the vessels. For this process, after applying Krebs solution containing 20 mM potassium chloride (KCI), phenylephrine (Phe) was added at a concentration of 10⁻⁷ M. This process was repeated 3 times in succession. After the vitalization procedure, the vessels were washed with Krebs solution and an additional 30-minute resting period was started. To investigate whether the endothelial layer lining the inner surface of the vessel lumen is healthy after the resting period, after adding phenylephrine at a concentration of 10⁻⁶ M to the baths, acetylcholine (ACh) at a concentration of 10⁻⁶ M was added, and the relaxation response was calculated as a percentage value. Vessels with a relaxation response of 60% or more were accepted as undamaged endothelium, and those below 60% were excluded from the experimental protocol. The contraction and relaxation signal responses received from the vessels were amplified by the amplifier, and then transferred to the software program via the data acquisition device.

After determining the vessels to be included in the experimental protocol, the contraction and relaxation responses of the vessels were examined with the following protocols.
1- Contractile response protocols:
   a) Potassium chloride (KCl)-mediated contractile response protocol: By adding 20, 40, and 80 mM KCI to the organ bath chamber, respectively, the contractile responses of the vessels were obtained.
   b) Phenylephrine (Phe)-mediated contractile response protocol: Increasing doses of Phe were added to the bath fluid cumulatively (10⁻⁹ - 3x10⁻⁵ M) and contractile responses were obtained.
2- Relaxation response protocols:
   a) Acetylcholine (ACh) mediated relaxation response (endothelium dependent relaxation) protocol: Following contraction of the vessels with a submaximal dose of Phe (10⁻⁶ M), the cumulative relaxation responses to ACh doses (10⁻⁹ - 3x10⁻⁵ M) were recorded and relaxation responses were obtained.
   b) Sodiumnitroprusside (SNP)-mediated relaxation response (endothelium-independent relaxation) protocol: Following contraction of the vessels with a submaximal dose of Phe (10⁻⁶ M), the cumulative relaxation responses to SNP doses (10⁻¹⁰ - 3x10⁻⁴ M) were recorded and relaxation responses were obtained.

The results were evaluated using a statistical program. When statistically evaluated, two-way analysis of variance (two-way ANOVA) and Bonferroni posthoc test were used in order to illustrate the multiple-group comparison of dose-dependent vascular response data. The p value for statistical significance was taken as p<0.05. Values are given as mean ± standard error (SEM).

### Thoracic Aorta Responses

1- Contractile response protocols:
   - Potassium chloride (KCI) mediated protocol: When comparing KCI dose-response curves at 20, 40 and 80 mM concentrations; no differences were observed between the control, SHAM, aurantiamide acetate, and hypertension groups (p>0.05). However, the contractile responses of the hypertension+aurantiamide acetate group at increasing KCI concentrations were observed to be significantly lower compared to the other groups (p<0.001).
   - Phe-mediated protocol: When comparing the dose-response curves of increasing dose Phe; no differences were observed between the control, SHAM, and aurantiamide acetate groups (p>0.05). On the other hand, Phe-mediated contraction responses were significantly increased in the hypertension group compared to the control group (*, p<0.05 and **, p<0.01). Contractile responses from the hypertension+aurantiamide acetate group were significantly reduced compared to the hypertension group (#, p<0.05 and ###, p<0.001). Aurantiamide acetate treatment resulted in improvement in Phe-mediated contractile responses, which were impaired in the hypertension group.
2- Relaxation response protocols:
   ACh-mediated protocol (endothelium-dependent relaxation responses): When comparing the dose-response curves of ACh at increasing dose, no difference was observed between the relaxation responses of the control, SHAM, and aurantiamide acetate groups (p>0.05). A statistically significant decrease was observed in the vasodilation response in response to ACh in the hypertension group compared to the control group (*, p<0.05; **, p<0.01, and ***p<0.001). In the hypertension+aurantiamide acetate group, with the administration of treatment, it was observed that there was an improvement in ACh-mediated relaxation responses, which were observed to deteriorate in hypertension (#, p<0.05 and ##, p<0.01).
   - SNP-mediated protocol (endothelium-independent relaxation responses): When comparing dose-response curves of increasing dose SNPs; no statistical difference was observed between the relaxation responses of the control, SHAM, and aurantiamide acetate groups (p>0.05). A significant decrease was observed in the smooth muscle-mediated relaxation response of the hypertension groups compared to the control group (**, p<0.01 and ***, p<0.001). However, there was no difference in SNP response in the hypertension+aurantiamide acetate group compared to the hypertension group (p>0.05).

As a result of examining the changes in body weight, blood pressure values, erythrocyte deformability, vascular isolation and vascular tone in the experimental groups during the experimental period, it has been determined that aurantiamide acetate has an endothelium-mediated vascular function-regulating effect, thereby reducing the complications of hypertension and cardiovascular morbidity in hypertension patients. In addition, as a result of the analyzes made, it was observed that aurantiamide acetate increased the deformability of erythrocytes and by means of this increase, erythrocytes could change their shape while passing through small vessels. Since erythrocytes can change their shape and pass through the vessels more easily in this way, aurantiamide acetate provides an increase in blood fluidity by reducing blood viscosity. By means of the specified properties, aurantiamide acetate is used in the treatment of hypertension. Therefore, a drug containing aurantiamide acetate of the present invention is indicated in the treatment of hypertension.

### REFERENCES

[1] Acibadem Web and Medical Editorial Board. (2021b, May 10). Hypertension. Acibadem Saǧlik Grubu, https://www.acibadem.com.tr/ilqi-alan i/h i pertansivon/#tedavi-vontem leri
[2] Aurantiamit asetat | GreenMolBD. (2021). GreenmolBD. https://www.qreenmolbd.qov.bd/compound/115
[3] Yoon, C. S., Kim, D. C., Lee, D. S., Kim, K. S., Ko, W., Sohn, J. H., Yim, J. H., Kim, Y. C., & Oh, H. (2014). Anti-neuroinflammatory effect of aurantiamit asetat from the marine fungus Aspergillus sp. SF-5921: Inhibition of NF-KB and MAPK pathways in lipopolysaccharide-induced mouse BV2 microglial celis. International Immunopharmacology, 23(2), 568-574. https://doi.org/10.1016/j.intimp.2014.10.006
[4] Chen, L., Liu, Y., Jia, D., Yang, J., Zhao, J., Chen, C., Liu, H., & Liang, X. (2016). Pharmacokinetics and Biodistribution of Aurantiamit and Aurantiamit Asetat in Rats after Oral Administration of Portulaca oleracea L. Extracts. Journal of Agricultural and Food Chemistry, 64(17), 3445-3455. https://doi.org/10.1021/acs.iafc.6b00470

## Claims

1. Aurantiamide acetate for use in the treatment of hypertension.

2. Antihypertensive drug containing aurantiamide acetate for use in the treatment of hypertension.

3. A drug for use according to Claim 2, **characterized in that**, said drug is administered orally.

4. A drug for use according to Claim 2, **characterized in that**, the drug is administered intraperitoneally (i.p.) at a dose of 0.5 mg/kg (weight of aurantiamide acetate/body weight) per day for 5 days per week over 4 weeks, resulting in a cumulative weekly dose of 2.5 mg/kg (weight of aurantiamide acetate/body weight).

## Patentansprüche

1. Aurantiamidacetat zur Verwendung bei der Behandlung von Bluthochdruck.

2. Blutdrucksenkendes Arzneimittel, das Aurantiamidacetat enthält, zur Verwendung bei der Behandlung von Bluthochdruck.

3. Arzneimittel zur Verwendung nach Anspruch 2, **dadurch gekennzeichnet, dass** das Arzneimittel oral verabreicht wird.

4. Arzneimittel zur Verwendung nach Anspruch 2, **dadurch gekennzeichnet, dass** das Arzneimittel intraperitoneal (i.p.) in einer Dosis von 0,5 mg/kg (Gewicht von Aurantiamidacetat/Körpergewicht) pro Tag an 5 Tagen pro Woche über 4 Wochen verabreicht wird, was zu einer kumulativen Wochendosis von 2,5 mg/kg (Gewicht von Aurantiamidacetat/Körpergewicht) führt.

## Revendications

1. Acétate d'aurantiamide destiné au traitement de l'hypertension.

2. Médicament antihypertenseur contenant de l'acétate d'aurantiamide destiné au traitement de l'hypertension.

3. Médicament destiné à être utilisé selon la revendication 2, **caractérisé en ce que** ledit médicament est administré par voie orale.

4. Médicament destiné à être utilisé selon la revendication 2, **caractérisé en ce que** le médicament est administré par voie intrapéritonéale (i.p.) à une dose de 0.5 mg/kg (poids de l'acétate d'aurantiamide/poids corporel) par jour pendant 5 jours par semaine durant 4 semaines, ce qui correspond à une dose hebdomadaire cumulative de 2.5 mg/kg (poids de l'acétate d'aurantiamide/poids corporel).
